# EUROPEAN PATENT APPLICATION

(11) **EP 2 389 873 A2**
(43) Date of publication of application: **30.11.2011**
(21) Application number: 11250549.0
(22) Date of filing: 25.05.2011
(51) Int. Cl.: A61B 17/03

(54) **Surgical fastener and drive instrument for soft tissue**

(30) Priority: 26.05.2010 US 348356 P; 29.03.2011 US 74113
(71) Applicant: Tyco Healthcare Group LP, New Haven, CT 06511 (US)
(72) Inventor: Glick, Jonathan P., Hamden, CT 06518 (US); Lehn, Randolph F., Stratford, CT 06614 (US)
(74) Representative: Soames, Candida Jane

(57) **Abstract**

A surgical fastener (10) for delivering into body tissue during a surgical procedure is disclosed. The surgical fastener (10) includes a continuous helical coil (40) having a proximal end and a distal end (21). The continuous helical coil (40) has a cross-section with at least five sides. A first helical coil section (42) is located at the distal end (21). A tapered point (22) extends from the first helical coil (42). A central passage (30) that passes through the center of the fastener (10) has a non-circular shape. The central passage (30) defines a longitudinal axis ("X"). The continuous helical coil (40) has a profile transverse to the longitudinal axis ("X"). The profile is non-circular. A tail (60) is located at the proximal end of the continuous helical coil (40).

## Description

### CROSS REFERENCE TO RELATED APPLICATION

The present application claims the benefit of and priority to U.S. Provisional Application Serial No. 61/348,356, filed on May 26, 2010, the entire contents of which are incorporated herein by reference.

### BACKGROUND

### 1. Technical Field

This application generally relates to surgical fasteners and their associated applicators, and more particularly, to surgical fasteners and related applicators for surgically fastening material onto tissue, such as for a hernia repair using mesh.

### 2. Background of Related Art

Surgical fasteners are used to eliminate the need for suturing, which is often time consuming and inconvenient. Surgical fasteners accomplish in seconds what would have taken many minutes to accomplish by suturing, thus reducing operating time and trauma to the patient. In hernia repair procedures, for example, the weakened area of the abdominal wall may be reinforced with a synthetic mesh. In such an instance, a surgical fastener in the form of an helical fastener may be used, in lieu of, or in addition to, a surgical suture to fix the position of the mesh.

Helical fasteners rotated into tissue are well known in the surgical art. Such fasteners have a threadform in the shape of a helical coil or thread and may have a central reinforcing shaft or a head. Torque may be applied to the faster at the head or by torque transmitting features located either on the outside radial surface of the fastener or head or the inside surface of the fastener in the instance of a cannulated fastener.

In view of the foregoing it can be appreciated that a need exists for improved surgical fasteners and related instruments for their application.

### SUMMARY

The present disclosure is directed to a surgical fastener for delivery into body tissue during a surgical procedure. The surgical fastener includes a helical fastener having a proximal end and a distal end. The threadform of the helical fastener may be a coil or thread having a cross-section with at least three sides and preferably five sides. Throughout this specification, aspects of a first embodiment discussed as a coil may be extended to a second embodiment having a thread and vice versa.

A first helical coil section is located at the distal end. A tapered point extends from the first helical coil. A central passage that passes through the center of the fastener has a non-circular shape. The central passage defines a longitudinal axis. The continuous helical coil has a profile transverse to the longitudinal axis. The profile is non-circular and may be tri-lobal. A tail is located at the proximal end of the continuous helical coil. The tail may incorporate a feature to entrap and restrain the mesh against tissue.

The non-circular central passage may have a shape in the form of either a D, oval, elliptical, square, triangular, or another polygonal shape.

The head includes an angle Θ as defined by a leading face of the head and a proximal surface of the cross-section. The leading face of the fastener has a triangular shape. The angle Θ is greater than 50°. The tail includes an angle Φ as defined by a trailing face of the tail and a proximal surface of the cross-section. The angle Φ is greater than 110°.

The cross-section has at least a top side and a bottom side that are substantially parallel. The cross-section has a perpendicular side that is substantially perpendicular to the parallel top and bottom sides. The cross-section has a leading side that forms an angle Ω with the longitudinal axis and a trailing side that forms angle Σ with the longitudinal axis. Both angle Ω and angle Σ are internal angles formed by the triangle defined by the leading side, the trailing side, and the longitudinal axis. The angle Ω is typically less than the angle Σ, although the possible ranges of angles Ω and Σ may overlap. Angle Σ may approach 90°, allowing for a small molding draft angle.

### BRIEF DESCRIPTION OF THE DRAWINGS

The accompanying drawings, which are incorporated in and form part of the specification, illustrate the present disclosure when viewed with reference to the description, wherein:

FIG. 1 depicts a side elevational view of a helical fastener of an embodiment the present disclosure;

FIG. 2 depicts a first end view of the helical fastener of FIG. 1;

FIG. 3 depicts a perspective view of a second end of the helical fastener of FIGS. 1 and 2;

FIG. 4 depicts a further side elevational view of a helical fastener of FIGS. 1-3;

FIG. 5 depicts a cross-sectional view of the helical fastener of the present disclosure as taken through 5-5 of FIG. 4;

FIG. 6 depicts a cross-sectional view of the helical fastener of FIGS. 1-5 as taken through 6-6 of FIG. 1;

FIG. 7 depicts a schematic side cross-sectional view of an applicator of the present disclosure;

FIG. 8 is a schematic cross-sectional end view of a terminal end of the applicator, illustrating a plurality of helical fasteners loaded therein;

FIG. 9 is a schematic cross-sectional view of the terminal end of the applicator, as take through 9-9 of FIG. 7, illustrating one embodiment of the drive rod;

FIG. 10 is perspective view of an embodiment of a drive rod of the present disclosure, illustrating a flattened circular shaped transverse cross-sectional profile;

FIG. 11 is perspective view of another embodiment of a drive rod of the present disclosure, illustrating a D-shaped transverse cross-sectional profile;

FIG. 12 is perspective view of yet another embodiment of a drive rod of the present disclosure, illustrating a circular shaped transverse cross-sectional profile with longitudinally extending recesses;

FIG. 13 is perspective view of still another embodiment of a drive rod of the present disclosure, illustrating a square shaped transverse cross-sectional profile with rounded comers; and

FIG. 14 is a schematic cross-sectional end view of another embodiment of the terminal end of the applicator of the present disclosure.

FIG. 15 depicts a side elevational view of a helical fastener of an additional embodiment of the present disclosure;

FIG. 16 depicts an end view of the helical fastener of FIG. 15;

FIG. 17 depicts a side elevational view of a helical fastener of a further embodiment of the present disclosure;

FIG. 18 depicts an additional side elevational view of the helical fastener of FIG. 17;

FIG. 19 depicts a side elevational view of a helical fastener of FIGS. 1-3 modified with a shaft;

FIG. 20 depicts a perspective end view of the helical fastener of FIG. 19;

Other features of the present disclosure will become apparent from the following detailed description, taken in conjunction with the accompanying drawings, which illustrate, by way of example, the principles of the present disclosure.

### DETAILED DESCRIPTION OF THE EMBODIMENTS

Embodiments of the presently disclosed fastener and fastener application for delivering the fastener into body tissue during a surgical procedure are described in detail with reference to the drawings, in which like reference numerals designate identical or corresponding elements in each of the several views. As used herein, the term "distal" refers to that portion of the surgical suture instrument, or component thereof, farther from the user while the term "proximal" refers to that portion of that portion of the surgical stapler or component thereof, closer to the user.

As is shown in the drawings, which are included for purposes of illustration and not by way of limitation, a continuous helical fastener and an applicator therefore are disclosed. The helical fastener has a high retentive surface area and the applicator has a simple design and functions to dispense the helical fasteners, without substantially deforming the fasteners, into body tissue, access to which is from one direction only.

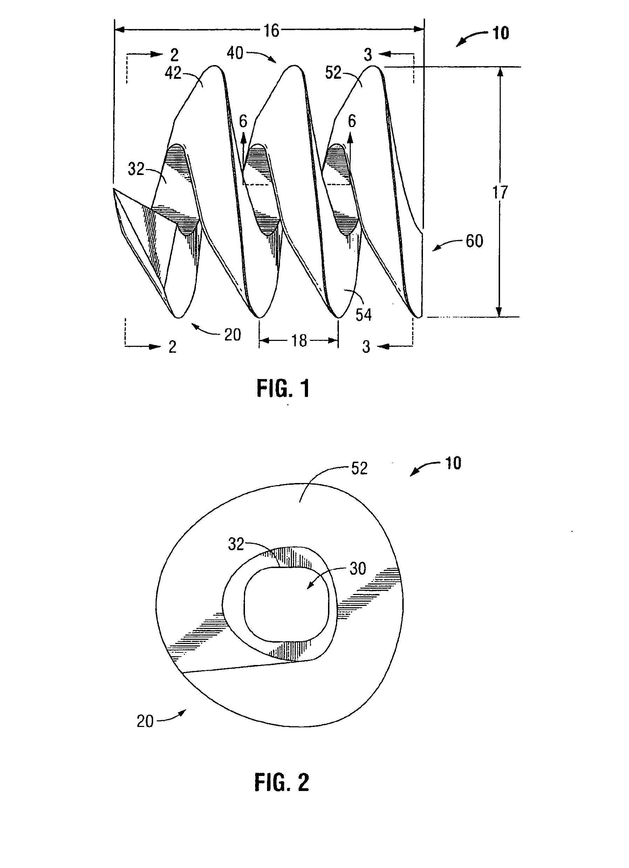

Referring now to the drawings, wherein like reference numerals identify identical or similar structural elements of the subject device throughout the several views, there is illustrated in FIGS. 1-6, a fastener, designated generally by reference numeral 10. Fastener 10 includes a tip or head portion 20, a helical coil body portion 40, and a tail portion 60.

The fastener 10 is formed into the configuration of a continuous helix and, as seen in FIG. 1, may have a depth 16, a diameter 17, and a pitch 18. The continuous helix may be longitudinally collapsible and expandable. In a particular application such as mesh anchoring for hernia repair, the pre-formed pitch can be about 0.050 inches. However, the pre-formed pitch can vary from about 0.0 inches to a maximum of approximately 3.0 times the coil height. It is contemplated that the pitch 18 may vary along the length of the fastener 10 to optimize the retaining force of the fastener 10.

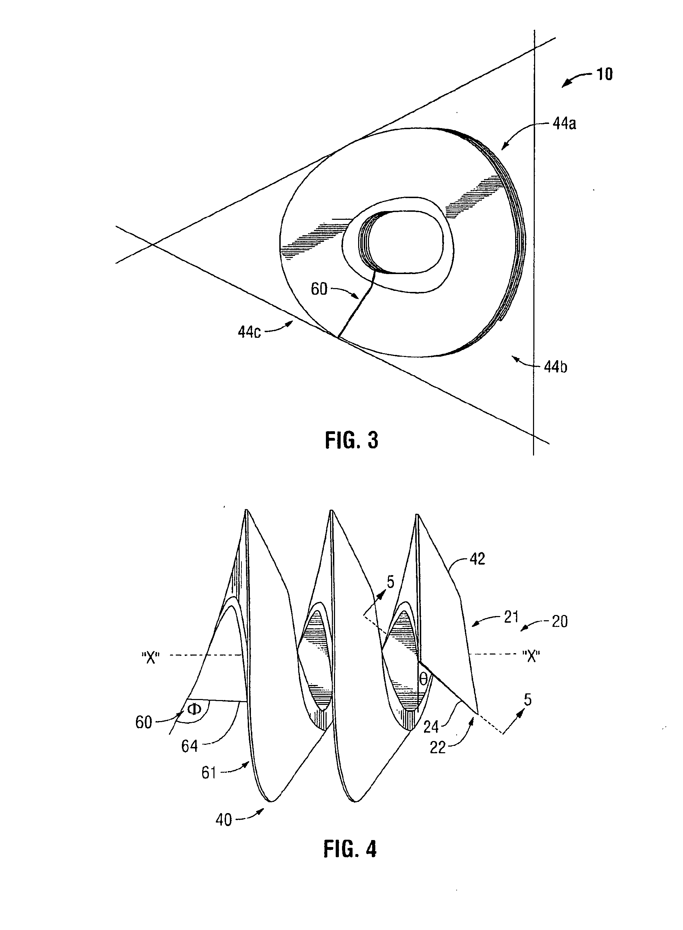

As seen in FIGS. 1 and 4, the tip or head portion 20 of the helical fastener 10 terminates in a distal-most or first coil 42 located at a distal end 21. Tip or head portion 20 terminates in a tapered point 22 extending from the first coil 42.

As seen in FIG. 2, the helical fastener 10 defines a center passage 30 and an internal surface 32. The center passage 30 receives a drive member (not shown) therethrough and the internal surface 32 transmits an applied rotational force to the helical fastener 10 from the drive member. The central passage 30 passes through the center of the fastener 10 and has a non-circular profile. The non-circular central passage 30 may have a shape in the form of either D-shaped, oval, elliptical, rectangular, triangular, or any combination thereof. The central passage 30 defines a longitudinal axis "X".

As shown in FIG. 3, the continuous helical coil 40 has a non-circular outer profile relative to the longitudinal axis "x". The outer profile is shown as being tri-lobal or having three lobes or rounded projections 44a, 44b, and 44c.

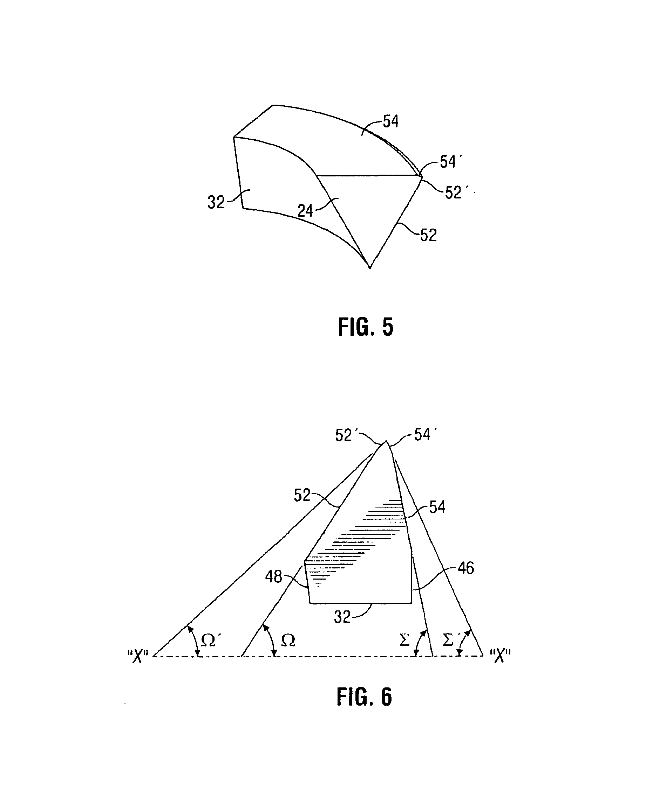

As seen in FIG. 4, the tip or head portion 20 defines an angle Θ between a leading face 24 of the tip or head portion 20 and a proximal surface 61 of tip or head portion 20. As shown in FIG. 5, the leading face 24 of the fastener 10 has a substantially triangular shape. The angle Θ is greater than approximately 50°.

As shown in FIG. 6, a cross-section of the helical coil 40 has at least a top side 46 and a bottom surface 48 that are substantially parallel to one another and extend from internal surface 32, defining center passage 30. The internal surface 32 is substantially parallel to the longitudinal "X" axis.

The cross-section helical coil 40 has a leading surface 52 that forms an angle Ω with the longitudinal axis "x" and a trailing surface 54 that forms angle Σ with the longitudinal "X" axis. Both angle Ω and angle Σ are internal angles formed by the triangle defined by the leading surface 52, the trailing surface 54, and the longitudinal "X" axis. The angle Ω is less than the angle Σ. The angle Ω is about 60° and the angle Σ is about 85°, but can approach 90° with allowance for a molding draft angle.

Leading surface 52 and trailing surface 54 meet in a tapered point consisting of leading tip surface 52' and trailing tip surface 54'. Leading tip surface 52' is the outer most extension of leading surface 52, and trailing tip surface 54' is the outer most extension of the trailing surface 54. Leading tip surface 52' and trailing tip surface 54' form respective angles Ω' and angle Σ' with the longitudinal "X" axis. Angles Ω' and angle Σ' are respectively less than angles Ω and angle Σ.

As seen in FIG. 4, the tail portion 60 includes an angle Φ as defined by a trailing face 64 of the tail portion 60 and a proximal surface or the top side 46 of the cross-section. The angle Φ is greater than approximately 110°.

It is contemplated that the fastener be made from a metal, a plastic, or an absorbable material. Examples of materials that can be used in constructing the helical fastener 10 include titanium, titanium alloys, stainless steel, nickel, chrome alloys and any other biocompatible implantable metals. Other options for materials are liquid crystal polymers, HDPE, polyglycolic acid, and polyglycolid hydroxgacetic acid. Further, it may also be desirable to coat the fastener, or a portion thereof, with a biocompatible lubricious material that provides for easier delivery of the fastener into tissue. Various combinations of materials, such as polymer alloys and or coatings are contemplated.

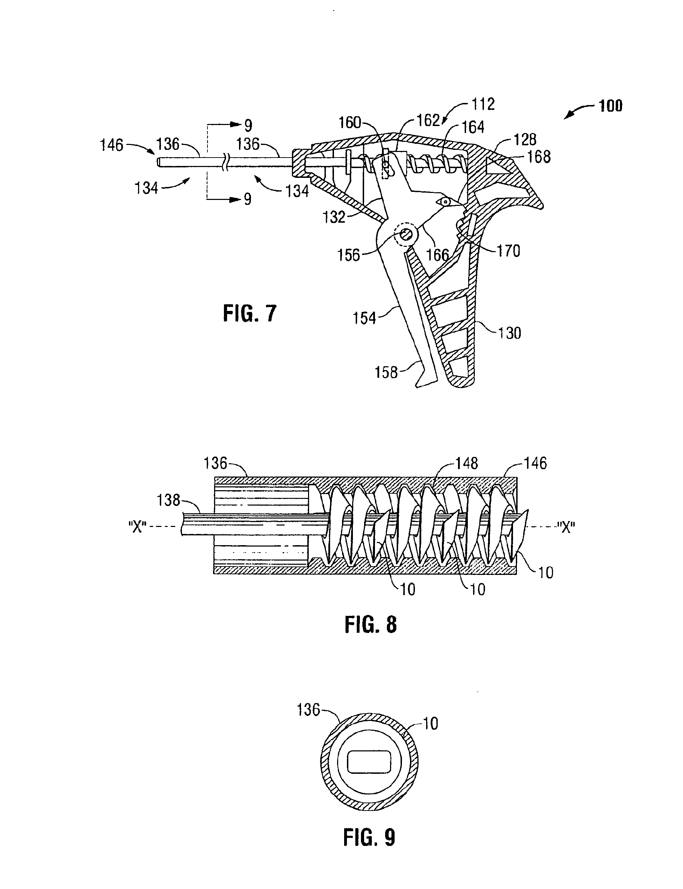

With reference to FIGS. 7-9, a helical fastener 10 is attached to tissue by employing a novel applicator 100 which rotates the fastener 10 into tissue. The dimensions and physical characteristics of the helical fastener 10 are selected to insure a secure attachment of the fastener 10 to tissue. Similarly, the dimensions and physical characteristics of the applicator 100 utilized to dispense the fasteners 10 into tissue are dependent upon the application.

With continued reference to FIG. 7, the applicator 100 comprises a proximal portion 128 having a handle 130 and an actuator 132 and a cooperating elongated distal portion or cannula 134 housing a plurality of fasteners. In general, through the manipulation of the actuator 132, the fasteners are ejected, one by one, out of a distal portion 134 and into body tissue. The applicator 100, hereinafter described in more detail, is equally proficient in driving each of the embodiments of fasteners set forth above into tissue.

The distal portion 134 includes an outer tube 136 housing a drive rod 138. It is to be appreciated that the drive rod 138 may receive a single fastener or a plurality of fasteners.

Turning to FIGS. 8, the outer tube 136 is formed with internal threads 148, which operate to engage and eject a fastener 10. As shown, the internal threads 148 are machined or formed solely within the inside of the outer tube. The terminal end of the applicator is effective with a relatively small overall outer diameter, i.e., on the order of 5 mm.

In order to eject surgical fasteners from the distal portion 134, the actuator 132 functions to turn the drive rod 138. As the rotator turns, the distal end 21 of a fastener is threaded out of the threads 148 of the applicator 100.

A lever 154 is pivotally connected about a midpoint 156 to the handle 130. A first end 158 of the lever 154 is to be configured for gripping by hand. A second end 160 of the lever is to be adapted to pivotally engage a nut driver 162.

The nut driver 162 of the applicator 100 travels upon a high helix lead screw 164, which is rotatably mounted within the proximal portion 128. As shown, a longitudinal axis of the high helix lead screw 164 is coaxial with the longitudinal axis "x" extending through the distal portion 134 of the applicator 100. Upon manipulation of the lever 154, the nut driver 162 travels along the lead screw 164 causing it to rotate through a connection of the lead screw 164 to the drive rod 138. The action of the lead screw 164 causes the rotator to rotate. The lead screw 164 may be connected to the drive rod 138 by any conventional means. For instance, the lead screw 164 can have an internal bore receiving and engaging an end of the drive rod 138. Further, the length of travel of the nut driver 162 along the lead screw 164 is chosen such that it causes the rotator to rotate a predetermined number of times so that a single helical fastener 10 is ejected from the applicator 100.

Additionally, as shown, the lever further comprises a midsection extension 166. Pivotally attached to the midsection extension 166 of the lever 154 is contemplated to be a spring loaded pawl 168 adapted to releasably engage gear teeth 170 formed in the interior of the handle 130. Spring loaded pawl 168 is configured to prohibit the lever 154 from backstroking until it has been completely depressed. Upon complete depression of the lever 154, the pawl 168 clears the gear teeth 170 and the spring biasing the pawl 168 rotates the pawl 168 away from the teeth 170, thereby allowing the lever 154 to return to its undepressed condition.

In operation, upon complete depression of the lever 154, the nut driver 162 travels a pre-determined distance along the lead screw 164, causing the drive rod 138 to rotate a pre-determined number of revolutions corresponding to a number of turns of a particular helical fastener 10. As the drive rod 138 rotates, the fasteners 10 retained by the rotator also rotate and the coils of the most distal fastener 10 are threaded out of the terminal end 146 of the applicator 100 and into tissue. Moreover, where the lever 154 is only partially depressed, the spring loaded pawl 168 operates to hold the lever 154 stationery and will continue to function to hold the lever 154 stationery until the lever 154 has been completely depressed. In this way, the delivery of fasteners into body tissue is controlled so that only a single fastener may be completely ejected out of the applicator 100 and pressed into body tissue at a time.

As shown, the proximal portion 128 is fabricated to have a reusable handle that can be re-sterilized, and the distal portion is made disposable. Thus, upon discharge of all the fasteners 10 from distal portion 134, the distal portion would be discarded and replaced. The handle could be reused up to a limited number of procedures.

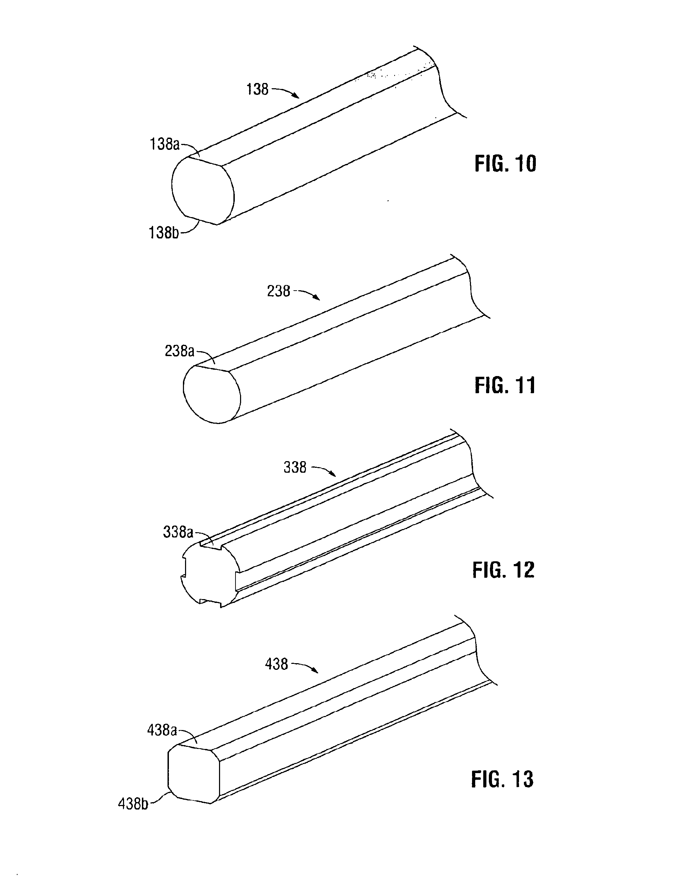

With reference to FIG. 10, the drive rod 138 has a substantially circular cross-sectional profile defining two flat sections 138a, 138b to engage inner walls 32 of the central passage 30 of fastener 10. As shown in FIGS. 11-13, the rotator 238, 338, 438 may take any shape that has structure functioning to engage a plurality of fasteners and to facilitate turning into tissue. With reference to FIG. 11, a drive rod 238 may have a substantially circular cross-section and a single flat section 238. With reference to FIG. 12, a drive rod 338 may have a series of recess 338a about the outer circumference of the drive rood 338. With reference to FIG. 13, a drive rod 438 may have multiple surfaces 438a, 438b and one of these surfaces 438a may be greater than another of these surfaces 438b.

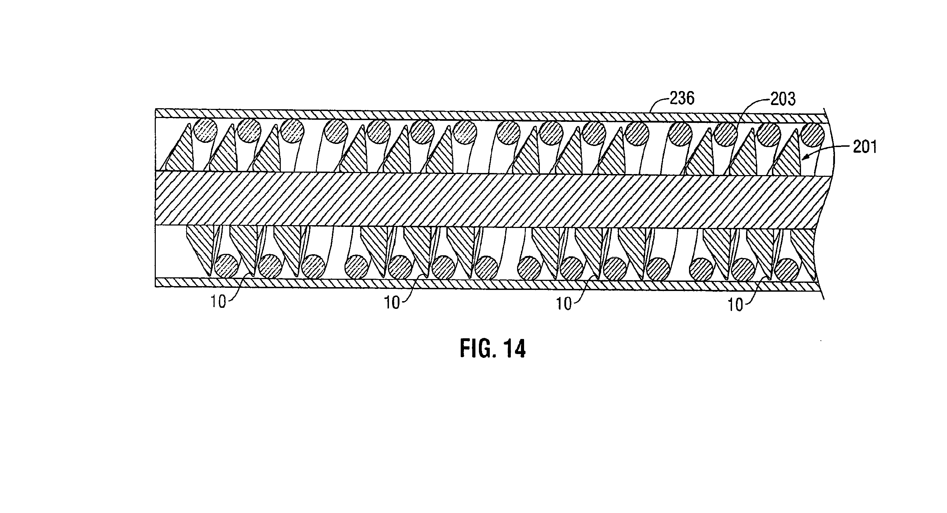

As shown in FIG. 14, the outer tube 136 is configured with a thread form 201 comprising an interlock spring 203 fixedly retained within the outer tube 136 and extending substantially the length thereof. The interlock spring 203 may be fixedly retained within the outer tube 136 by ensuring a tight interference between the parts or the interlock spring may be spot welded or equivalently bonded within the outer tube. The thread form 201 operates to guide the fasteners through the distal portion 134 and to eject them from the applicator 100.

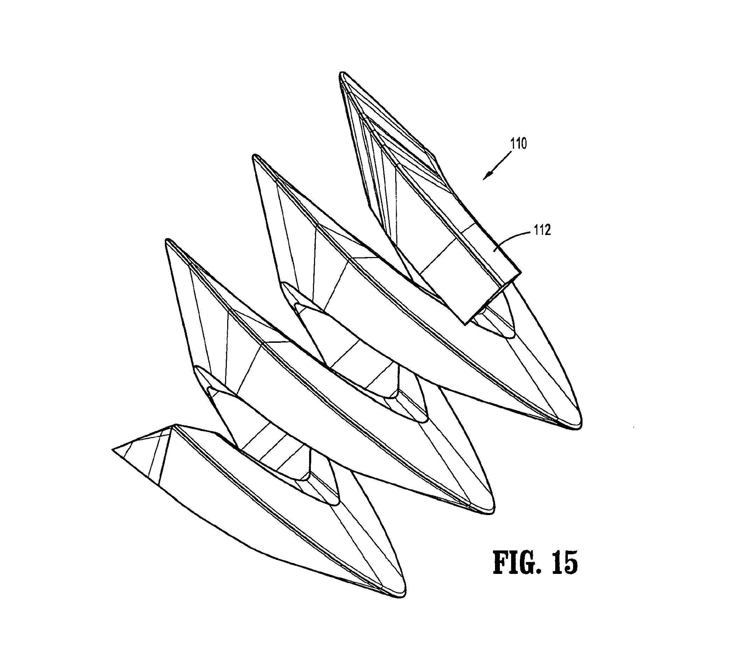

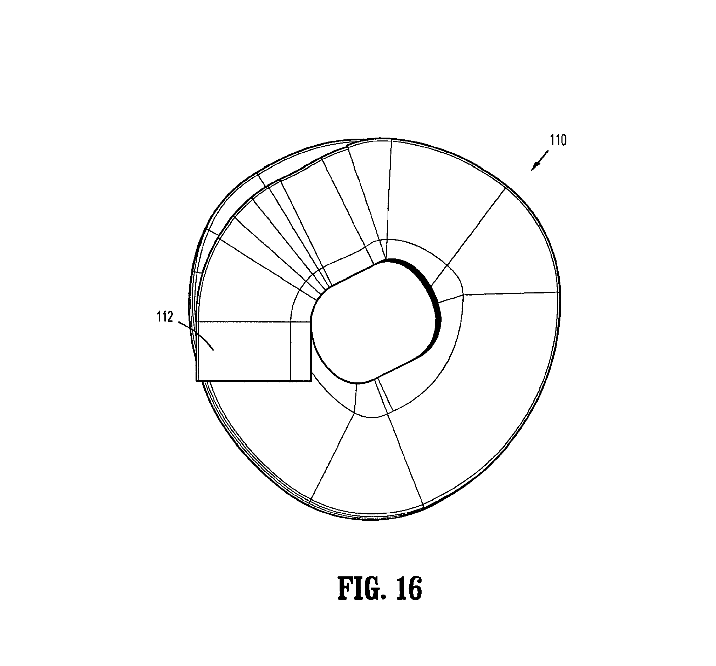

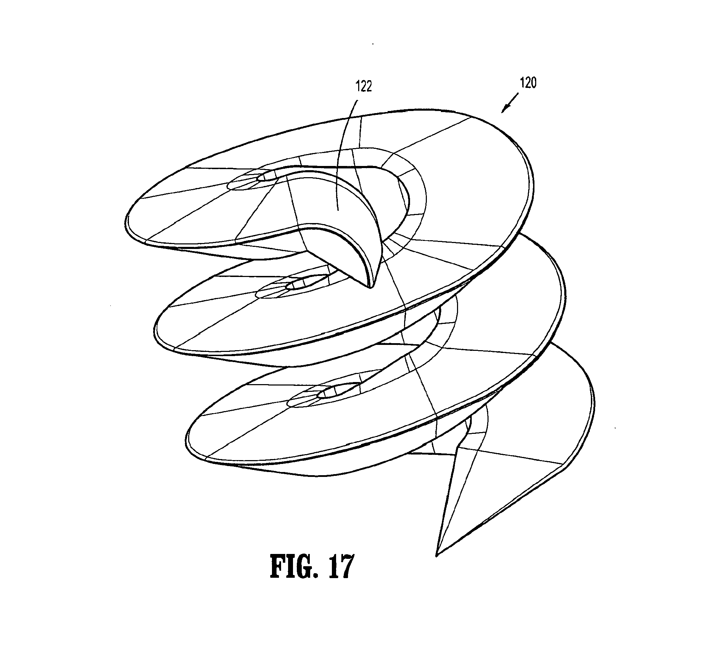

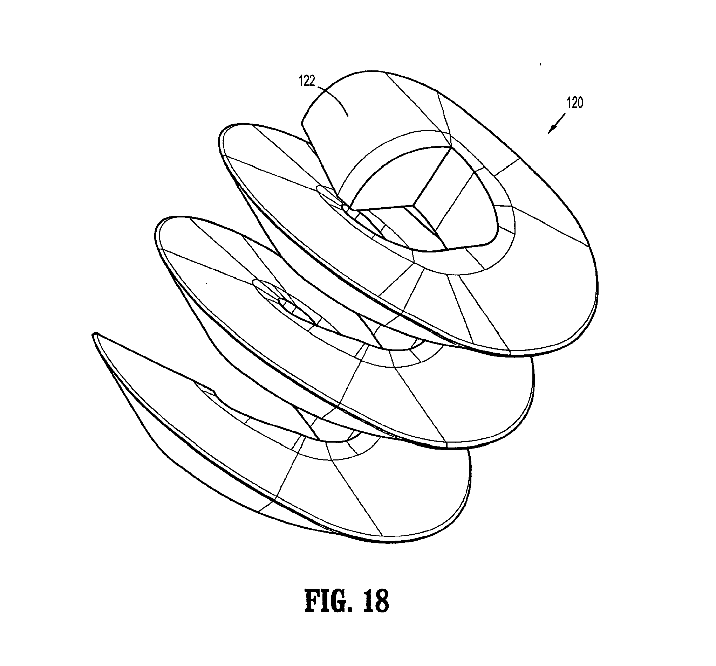

FIGS. 15 and 16 show an embodiment of a fastener 110 with a mesh stop 112 which decreases the gap to the adjacent coil to wedge the tissue and mesh within the gap and capture the mesh against the tissue. FIGS. 17 and 18 show a further embodiment of a fastener 120 with a mesh stop 122 which returns in the distal direction to capture the mesh against the tissue.

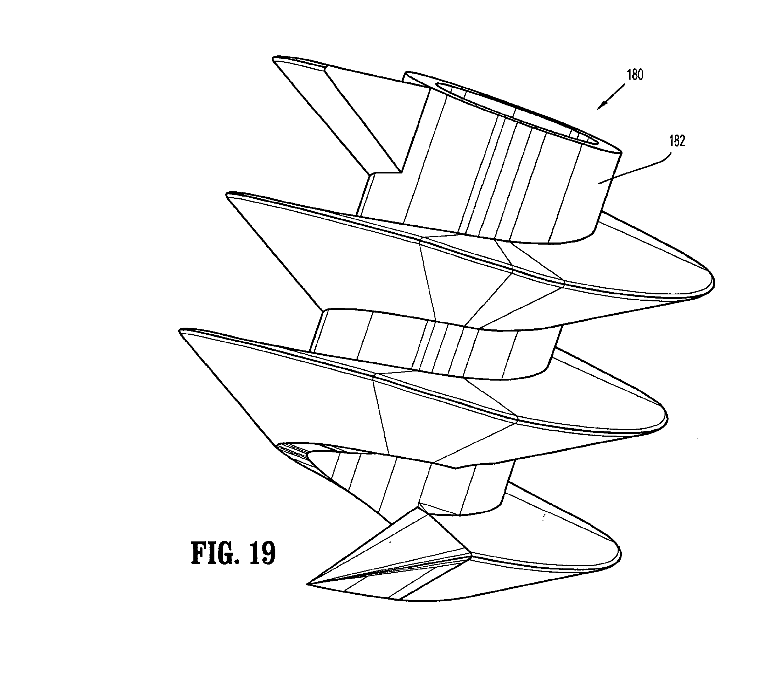

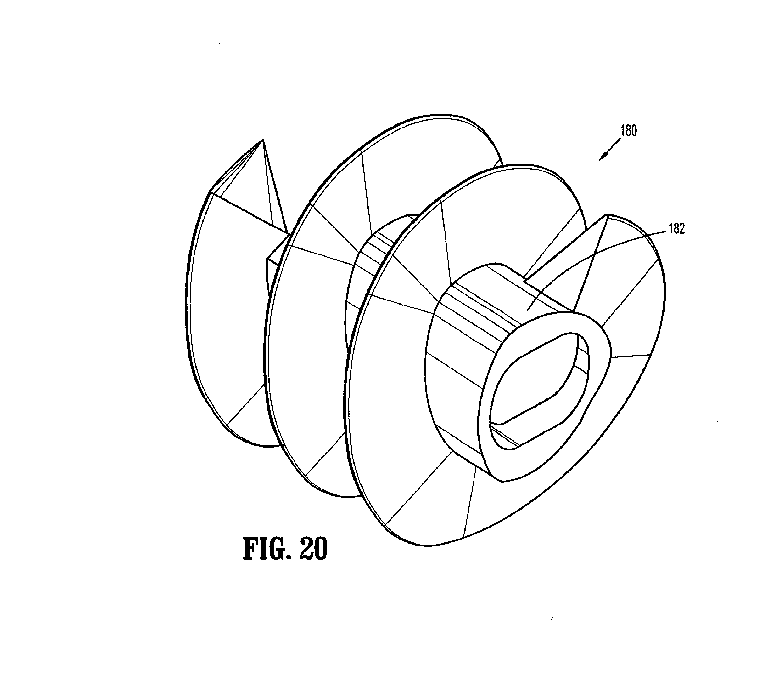

FIGS. 19 and 20 show a fastener 180 as in FIG. 1 modified with a central shaft 182 to reinforce the coils. Such a reinforcing shaft may be valuable when relatively weak materials, such as resorbable polymers are used to form the fastener.

From the foregoing, it will be appreciated that the helical fastener in applicator of the present disclosure functions to securely attach a fastener with high retentive surface area to tissue from one direction through the utilization of an applicator having a simple design. It is also to be appreciated that the present disclosure may be utilized in a number of applications including ligating tissue, hernia mesh repair, bladder neck suspension, and in conjunction with implant drug delivery systems or procedures involving positioning of surgical or implantable devices in patient.

While several particular forms of the disclosure have been illustrated and described, it will also be apparent that various modifications can be made without departing from the spirit and scope of the disclosure.

Thus, it should be understood that various changes in form, detail and application of the present disclosure may be made without departing from the spirit and scope of this disclosure.
The invention may be described by reference to the following numbered paragraphs:
1. A surgical fastener comprising a continuous helical coil having a distal end and a proximal end, wherein said continuous helical coil has a transverse cross-sectional profile defined by at least 5 surfaces, said continuous helical coil including a first helical coil segment adjacent to said distal end defining a tip portion, a tapered point defined at said distal end of said tip portion, a central passage extending through said helical coil, said central passage having a non-circular profile defining a longitudinal axis; and a tail portion located at said proximal end of said continuous helical coil.
2. The surgical fastener of paragraph 1, wherein said non-circular profile of said central passage includes a shape selected from a group consisting of D-shaped, oval, elliptical, square, and triangular.
3. The surgical fastener of paragraph 1, wherein said tapered point includes an angle Θ as defined by a leading face of said tapered point and a proximal surface of said cross-sectional profile, wherein said angle Θ is greater than approximately 50°.
4. The surgical fastener of paragraph 1, wherein said tail portion includes an angle Φ as defined by a trailing face of said tail portion and a proximal surface of said cross-sectional profile, wherein said angle Φ is greater than approximately 110°.
5. The surgical fastener of paragraph 1, wherein said non-circular profile has a tri-lobal shape.
6. The surgical fastener of paragraph 3, wherein said leading face has a triangular shape.
7. The surgical fastener of paragraph 1, wherein said cross-sectional profile has at least a top surface and a bottom surface that are substantially parallel.
8. The surgical fastener of paragraph 7, wherein said cross-sectional profile has a perpendicular surface that is substantially perpendicular to said parallel top and bottom surfaces.
9. The surgical fastener of paragraph 7, wherein said cross-sectional profile has a leading surface that forms an angle Ω with said longitudinal axis and a trailing surface that forms an angle ∑ with said longitudinal axis, wherein both angle Ω and angle Σ are internal angles formed by a triangle defined by said leading surface, said trailing surface, and said longitudinal axis.
10. The surgical fastener of paragraph 9, wherein said angle Ω is less than said angle Σ.

## Claims

1. A surgical fastener comprising:
a continuous helical coil having a distal end and a proximal end, wherein said continuous helical coil has a transverse cross-sectional profile defined by at least 5 surfaces;
said continuous helical coil including:
a first helical coil segment adjacent to said distal end defining a tip portion;
a tapered point defined at said distal end of said tip portion;
a central passage extending through said helical coil, said central passage having a non-circular profile defining a longitudinal axis; and
a tail portion located at said proximal end of said continuous helical coil.

2. The surgical fastener of claim 1, wherein said non-circular profile of said central passage includes a shape selected from a group consisting of D-shaped, oval, elliptical, square, and triangular.

3. The surgical fastener of claim 1 or claim 2, wherein said tapered point includes an angle Θ as defined by a leading face of said tapered point and a proximal surface of said cross-sectional profile, wherein said angle Θ is greater than approximately 50°.

4. The surgical fastener of any preceding claim, wherein said tail portion includes an angle Φ as defined by a trailing face of said tail portion and a proximal surface of said cross-sectional profile, wherein said angle Φ is greater than approximately 110°.

5. The surgical fastener of any preceding claim, wherein said non-circular profile has a tri-lobal shape.

6. The surgical fastener of claim 3, wherein said leading face has a triangular shape.

7. The surgical fastener of any preceding claim, wherein said cross-sectional profile has at least a top surface and a bottom surface that are substantially parallel.

8. The surgical fastener of claim 7, wherein said cross-sectional profile has a perpendicular surface that is substantially perpendicular to said parallel top and bottom surfaces.

9. The surgical fastener of claim 7, wherein said cross-sectional profile has a leading surface that forms an angle Ω with said longitudinal axis and a trailing surface that forms an angle ∑ with said longitudinal axis, wherein both angle Ω and angle Σ are internal angles formed by a triangle defined by said leading surface, said trailing surface, and said longitudinal axis.

10. The surgical fastener of claim 9, wherein said angle Ω is less than said angle Σ.
